# EUROPEAN PATENT APPLICATION

(11) **EP 4 647 082 A1**
(43) Date of publication of application: **12.11.2025**
(21) Application number: 24174797.1
(22) Date of filing: 08.05.2024
(51) Int. Cl.: A61K 38/00, A61K 38/12, A61K 9/00, C07D 513/04, C07K 7/06, A61P 25/06

(54) **PHARMACEUTICAL COMPOSITION COMPRISING A CGRP RECEPTOR ANTAGONIST FOR THE TREATMENT OF ACUTE MIGRAINE**

(71) Applicant: NUVIE BIO, Inc., San Diego, CA 92130 (US)
(72) Inventor: NEYER, Gebhard, Carlsbad (US); VERLANDER, Michael Stephen, Solana Beach (US); FINEMAN, Mark Stephen, San Diego (US); CADY, Roger Kenneth, Ozark (US); RIVIERE, Pierre Jean-Marie, San Diego (US)
(74) Representative: Reddie & Grose LLP

(57) **Abstract**

The present invention relates to a calcitonin gene-related peptide (CGRP) receptor antagonist of formula (I) or a or equivalent net peptide weight of a pharmaceutically active salt thereof, namely AXN-001, which is also known as FE 205030. A pharmaceutical composition comprising AXN-001 can be used in the treatment of acute migraine.

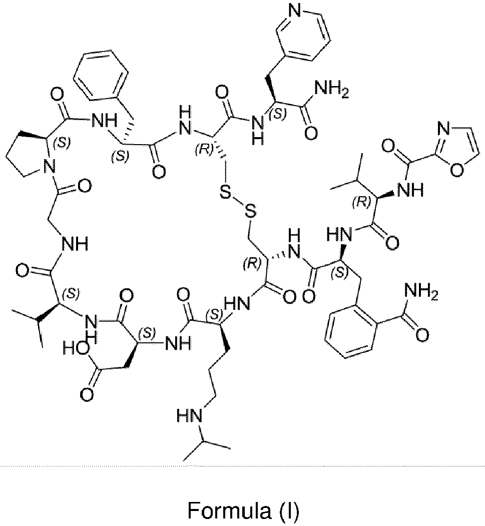

## Description

### Field of the Invention

The present invention relates to a calcitonin gene-related peptide (CGRP) receptor antagonist which may be used for the acute treatment of migraine and other CGRP-dependent conditions.

### Background

Migraine is a highly prevalent and disabling neurological disease, disproportionally impacting women. Migraine affects >1 billion people worldwide, more than >700 million of whom are women. In 2016, migraine was responsible globally for >45 million years of life lived with disability (YLDs).

Acute treatments for migraine, taken as needed during attacks, are the mainstay of migraine management. Most patients (>90%) on existing oral acute therapies report at least one unmet treatment need, in particular the needs for faster onset of action and higher efficacy.

Most frequently prescribed acute treatments of migraine include (i) previously approved triptans (small molecule agonists for serotonin 5-HT1B/1D receptors), and (ii) recently approved gepants (small molecule antagonists for the CGRP receptor). Triptans are more effective than gepants but associated with safety concerns (cardiovascular contraindications, medication overuse headache) and tolerability issues ("triptans sensations" characterized by chest pressure and difficulty to breathe). Whereas gepants are safer and better tolerated than triptans they are markedly less effective. Triptans are available as oral (PO), intranasal (IN), and subcutaneous (SC) products. Gepants are currently available only as oral tablet (ubrogepant, rimegepant) and nasal spray (zavegepant) products that are both marginally effective (i.e., -10% treatment effect on 2-hour pain freedom endpoint, with treatment effect being defined as % of drug responders minus % of placebo responders). There is currently no approved SC formulation of a CGRP receptor antagonist, most likely due to solubility or tolerability limitations.

Thus, there is a need for a pharmaceutical composition for the treatment of acute migraine which combines the advantages of triptans, i.e. rapid onset of action and high efficacy with the advantages of gepants, i.e. high safety and tolerability.

### Summary of the Invention

According to a first aspect of the invention, there is provided a pharmaceutical composition comprising 1 mg to 18 mg of a compound of formula (I) or pharmaceutically active salt thereof: 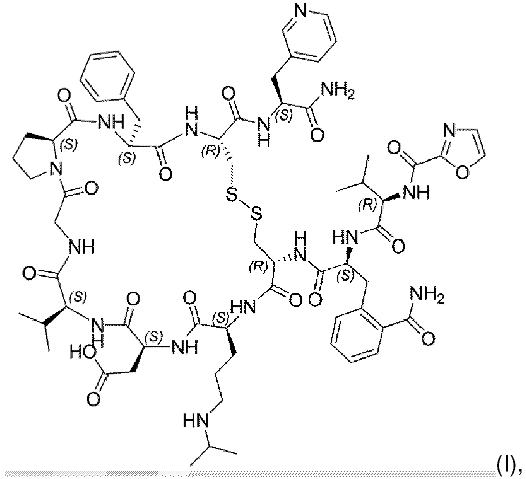 wherein the concentration of the compound of formula (I) or pharmaceutically active salt thereof is 2 mg/mL to 36 mg/mL.

The compound of formula (I) is also known as AXN-001 or FE 205030 and described in Srinivasan, et al. J Pharm Sci. 2022;111(1):247-261, hereby incorporated by reference.

WO 2016/110499 A1 is incorporated by reference.

The compound of formula (I) may also be represented by the name "Oxazole-2-carbonyl-D-Val-Phe(2-Cbm)-c(Cys-Orn (iPr)-Asp-Val-Gly-Pro-Phe-Cys)-3Pal-NH₂". 3Pal refers to 3-pyridylalanine. Phe(2-Cbm) refers to 3-(2-carbamoyl)phenylalanine.

Structurally, AXN-001 is a 11-mer monocyclic, branched peptide, which may be isolated as a hydrochloride salt. AXN-001 is a neuropeptide CGRP receptor antagonist, more specifically, AXN-001 is a highly potent, selective CGRP receptor antagonist having a combination of pharmacological properties which provide a rapid onset of action upon subcutaneous administration and with an ideal duration of response when delivered in a specific manner/composition, for example, between 3 and 8 hours.

Advantageously, the pharmaceutical composition comprising 1 mg to 18 mg of AXN-001 or a pharmaceutically active salt thereof, wherein the concentration of AXN-001 or a pharmaceutically active salt thereof is 2 mg/mL to 36 mg/mL supports an unprecedented target product profile (TPP), which addresses the unmet medical need, namely for patients and prescribers: speed of action, rapid return to function, and reduced recurrence, as well as safety, tolerability, efficacy, consistency, and lack of drug-drug interactions.

Advantageously, the combination of (i) the dose of AXN-001 or a pharmaceutically active salt thereof and (ii) the concentration of AXN-001 or a pharmaceutically active salt thereof provides for a differentiated and superior pharmacokinetic (PK) profile.

More specifically, formulations including 6 mg to 18 mg of AXN-001 or a pharmaceutically active salt thereof at a concentration of 12 mg/mL to 36 mg/mL may provide for faster, higher, and more consistent free plasma concentrations than any approved gepant. In turn, this provides for faster, higher, and more reliable efficacy than any approved gepant. More specifically, the pharmaceutical composition comprising a dose of 3 mg to 5 mg of AXN-001 or a pharmaceutically active salt thereof may provide an advantageous PK profile. Advantageously, such doses are fast acting and long acting. Advantageously, such doses achieve the desired plasma levels whilst requiring the minimum amount for efficacy.

The terms "pharmaceutical composition" and "composition" and "formulation" may be used interchangeably.

The term "pharmaceutically active salt thereof" includes, but shall not be limited to, pharmacologically active derivatives, *e.g.* esters, complexes, clathrates, *etc.*

The pharmaceutical composition is an injectable composition. An injectable composition may provide for subcutaneous or intravenous administration, for example, to a patient. Preferably, the composition is for subcutaneous administration.

Preferably, the pharmaceutical composition comprises the compound of formula (I) or an equivalent net peptide weight of a pharmaceutically active salt thereof. Herein, any weight provided in mg relating to AXN-001 is understood to mean the equivalent net peptide weight for the pharmaceutically active salt thereof. In other words, 6 mg AXN-001 recited in the claim is understood to refer, for a salt, to the equivalent net peptide weight to AXN-001 of the salt. The skilled person is readily aware that any dry peptide powder usually contains the peptides and other components, for example, water, absorbed solvents, counterions, and salts, which constitutes the gross peptide weight. In contrast, the net peptide weight is actual weight of the peptide component, which is commonly determined by amino acid analysis or Nitrogen analysis.

Preferably, the pharmaceutical composition comprises 4 mg to 18 mg of a compound of formula (I) or pharmaceutically active salt thereof, preferably 6 mg to 18 mg of a compound of formula (I) or a pharmaceutically active salt thereof. In the pharmaceutical composition, the concentration of the compound of formula (I) or a pharmaceutically active salt thereof is 8 mg/mL to 36 mg/mL, preferably 12 mg/mL to 36 mg/mL.

Advantageously, the pharmaceutical composition comprising a dose of 6 mg to 18 mg of a compound of formula (I) or a pharmaceutically active salt thereof may provide a rapid onset of action and a long duration, i.e. an advantageous PK profile. Advantageously, the pharmaceutical composition comprising a dose of 6 mg to 18 mg of a compound of formula (I) or a pharmaceutically active salt thereof may provide the formation of a depot.

Preferably, the pharmaceutical composition comprises 4 mg of a compound of formula (I) or a pharmaceutically active salt thereof. In the pharmaceutical composition, the concentration of the compound of formula (I) or a pharmaceutically active salt thereof is 8 mg/mL.

Preferably, the pharmaceutical composition comprises 8 mg of a compound of formula (I) or a pharmaceutically active salt thereof. In the pharmaceutical composition, the concentration of the compound of formula (I) or a pharmaceutically active salt thereof is 16 mg/mL.

Preferably, the pharmaceutical composition comprises 12 mg of a compound of formula (I) or a pharmaceutically active salt thereof. In the pharmaceutical composition, the concentration of the compound of formula (I) or a pharmaceutically active salt thereof is 24 mg/mL.

Without wanting to be bound by theory, the present inventors believe that the combination of 6 mg to 18 mg of AXN-001 or a pharmaceutically active salt thereof at a concentration (of AXN-001 or a pharmaceutically active salt thereof) of 12 mg/mL to 36 mg/mL administered subcutaneously advantageously creates a depot which results in longer duration of action. In turn, provision of a depot comprising AXN-001 or a pharmaceutically active salt thereof facilitates an improved pharmaceutical composition.

Preferably, the pharmaceutical composition comprises 1 mg to 6 mg of a compound of formula (I) or a pharmaceutically active salt thereof, preferably 3 mg to 5 mg of a compound of formula (I) or a pharmaceutically active salt thereof. In the pharmaceutical composition, the concentration of the compound of formula (I) or a pharmaceutically active salt thereof is 2 mg/mL to 12 mg/mL, preferably 6 mg/mL to 10 mg/mL.

Advantageously, the pharmaceutical composition comprising a dose of 3 mg to 5 mg of a compound of formula (I) or a pharmaceutically active salt thereof may provide an desirable PK profile. Advantageously, such doses are fast acting and long acting. Advantageously, such doses achieve the desired plasma levels whilst requiring the minimum amount for efficacy.

Preferably, the pharmaceutical composition comprises 1 mg of a compound of formula (I) or a pharmaceutically active salt thereof. In the pharmaceutical composition, the concentration of the compound of formula (I) or a pharmaceutically active salt thereof is 2 mg/mL.

Preferably, the pharmaceutical composition comprises 2 mg of a compound of formula (I) or a pharmaceutically active salt thereof. In the pharmaceutical composition, the concentration of the compound of formula (I) or a pharmaceutically active salt thereof is 4 mg/mL.

Preferably, the pharmaceutical composition comprises 4 mg of a compound of formula (I) or a pharmaceutically active salt thereof. In the pharmaceutical composition, the concentration of the compound of formula (I) or a pharmaceutically active salt thereof is 8 mg/mL.

Optionally, the pharmaceutical composition comprises 6 mg to 18 mg of AXN-001 or a pharmaceutically active salt thereof, for example, 7 mg to 17 mg, 8 mg to 16 mg, 9 mg to 15 mg, 10 mg to 14 mg, 11 mg to 13 mg, 12 mg, or any combination of the above endpoints. For example, the pharmaceutical composition may comprise at least 6 mg, at least 7 mg, at least 8 mg, at least 9 mg, at least 10 mg, at least 11 mg, at least 12 mg, at least 13 mg, at least 14 mg, at least 15 mg, at least 16 mg of AXN-001 or a pharmaceutically active salt thereof; for example, the pharmaceutical composition may comprise no more than 18 mg, no more than 17 mg, no more than 16 mg, no more than 15 mg, no more than 14 mg, no more than 13 mg, no more than 12 mg, no more than 11 mg, no more than 10 mg, no more than 9 mg of AXN-001 or a pharmaceutically active salt thereof; or any combination of the above endpoints.

Preferably, the composition comprises 8 mg to 16 mg of AXN-001 or a pharmaceutically active salt thereof.

Optionally, the pharmaceutical composition comprises a concentration of AXN-001 or a pharmaceutically active salt thereof of 16 mg/mL to 32 mg/mL, for example, 17 mg/mL to 31 mg/mL, 18 mg/mL to 30 mg/mL, 19 mg/mL to 29 mg/mL, 20 mg/mL to 28 mg/mL, 21 mg/mL to 27 mg/mL, 22 mg/mL to 26 mg/mL, 23 mg/mL to 25 mg/mL, 24 mg/mL, or any combination of the above endpoints. For example, the pharmaceutical composition may comprise at least 16 mg/mL, at least 17 mg/mL, at least 18 mg/mL, at least 19 mg/mL, at least 20 mg/mL, at least 21 mg/mL, at least 22 mg/mL, at least 23 mg/mL, at least 24 mg/mL, at least 25 mg/mL, at least 26 mg/mL, at least 27 mg/mL, at least 28 mg/mL, at least 29 mg/mL, at least 30 mg/mL, at least 31 mg/mL of AXN-001 or a pharmaceutically active salt thereof; for example, the pharmaceutical composition may comprise no more than 32 mg/mL, no more than 31 mg/mL, no more than 30 mg/mL, no more than 29 mg/mL, no more than 28 mg/mL, no more than 27 mg/mL, no more than 26 mg/mL, no more than 25 mg/mL, no more than 24 mg/mL, no more than 23 mg/mL, no more than 22 mg/mL, no more than 21 mg/mL, no more than 20 mg/mL, no more than 19 mg/mL, no more than 18 mg/mL, no more than 17 mg/mL of AXN-001 or a pharmaceutically active salt thereof; or any combination of the above endpoints.

Optionally, the pharmaceutical composition comprises a concentration of AXN-001 or a pharmaceutically active salt thereof, which is twice the dose per ml, for example 32 mg/ml for a 16 mg dose, *etc.*

Preferably, the pharmaceutical composition comprises 8 mg of AXN-001 or a pharmaceutically active salt thereof and the concentration of AXN-001 or a pharmaceutically active salt thereof is 16 mg/mL.

Preferably, the pharmaceutical composition comprises 16 mg of AXN-001 or a pharmaceutically active salt thereof and the concentration of AXN-001 or a pharmaceutically active salt thereof is 32 mg/mL.

The pharmaceutical composition is an injectable composition. An injectable composition may provide for subcutaneous or intravenous administration, for example, to a patient.

Optionally, the pharmaceutical composition is aqueous. For example, the pharmaceutical composition may comprise water, e.g. water for injection (WFI).

Optionally, the pharmaceutical composition has a pH between 4.5 to 5.5. For example, the pH of the pharmaceutical composition may be 4.5 to 5.5, 4.6 to 5.4, 4.7 to 5.3, 4.8 to 5.2, 4.9 to 5.1, or 5.0, or any combination of the above endpoints.

Advantageously, the pharmaceutical composition having a pH between 4.5 to 5.5 provides for reduced or no injection site reactions.

Optionally, the pharmaceutical composition comprises a buffering agent. Preferably, the buffering agent is sodium acetate trihydrate. Advantageously, the buffering agent maintains the pharmaceutical composition at the desired pH value or range. More specifically, the buffering agent modulates the rapid pH adjustment in the subcutaneous space and allows AXN-001 be absorbed rapidly prior to gel/depot due to neutral pH. This is particularly important because solubility of AXN-001 is low at neutral pH. In turn, the presence of a buffering agent such as sodium acetate trihydrate in the pharmaceutical composition is particularly important.

Optionally, the pharmaceutical composition comprises an isotonicity agent. Preferably, the isotonicity agent is mannitol. Advantageously, the isotonicity agent adjusts the tonicity of the pharmaceutical composition to be isotonic with blood. The presence of an isotonicity agent such as mannitol is particularly important for good tolerability.

Preferably, the pharmaceutical composition comprises 32.0 mg AXN-001, 1.36 mg sodium acetate trihydrate, 45.0 mg mannitol, hydrochloric acid q.s. to pH 5.0, sodium hydroxide q.s. to pH 5.0, and Water for injection to 1.0 mL.

According to a further aspect of the invention, there is provided a kit of parts comprising a pharmaceutical composition comprising 1 mg to 18 mg of a compound of formula (I) or a pharmaceutically active salt thereof: 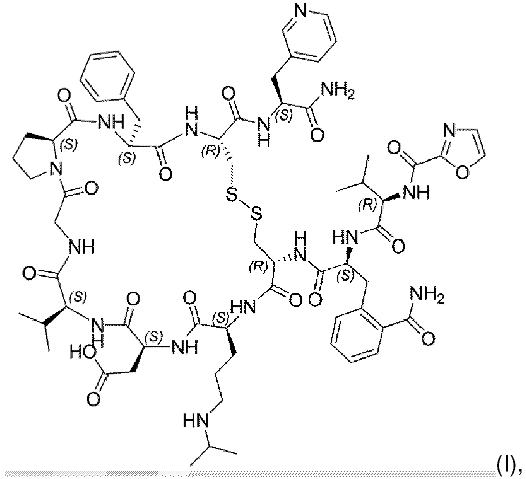 wherein the concentration of the compound of formula (I) or pharmaceutically active salt thereof is 2 mg/mL to 36 mg/mL.

The kit of parts may comprise a means for administration, for example, a means for injection. The kit of parts may comprise syringe, *e*.*g*. a 1 mL syringe, for comprising the pharmaceutical composition. The kit of parts may comprise an injector, *e*.*g*. an autoinjector, preferably a subcutaneous autoinjector, for comprising the pharmaceutical composition.

According to a further aspect of the invention, there is provided a pharmaceutical composition comprising 1 mg to 18 mg of a compound of formula (I) or a pharmaceutically active salt thereof: 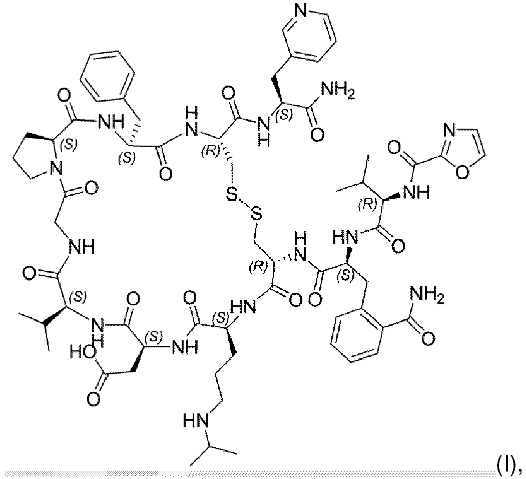 wherein the concentration of the compound of formula (I) or pharmaceutically active salt thereof is 2 mg/mL to 36 mg/mL for use in the treatment of acute migraine.

Acute migraine treatment is understood to mean relieving the migraine symptoms, in contrast to preventive treatment which is aimed at reducing the frequency and severity of the migraine events. Advantageously, the pharmaceutical composition for use in the treatment of acute migraine provides for patient well-being featured by a) rapid improvement of headache, b) relief of non-pain symptoms and c) absence of adverse events.

Optionally, the pharmaceutical composition is for use in the treatment of acute migraine in a patient in need thereof. The pharmaceutical composition may be for use in the treatment of acute migraine in a human patient or in a human subject.

Optionally, the pharmaceutical composition is administered subcutaneously, *i.e.* by subcutaneous injection. For example, the pharmaceutical composition is administered via subcutaneous (SC) autoinjector. Advantageously, this provides for a patient-friendly delivery.

Optionally, the pharmaceutical composition is for administration at a dose of 6 mg to 18 mg of AXN-001 or pharmaceutically active salt thereof per subject (per episode), for example, 7 mg to 17 mg, 8 mg to 16 mg, 9 mg to 15 mg, 10 mg to 14 mg, 11 mg to 13 mg, 12 mg, or any combination of the above endpoints. Preferably, the pharmaceutical composition is for administration at a dose of 8 mg to 16 mg per subject.

Preferably, the pharmaceutical composition is for administration at a dose of 1 mg AXN-001 or a pharmaceutically active salt thereof by subcutaneous administration. Preferably, the pharmaceutical composition is for administration at a dose of 2 mg AXN-001 or a pharmaceutically active salt thereof by subcutaneous administration. Preferably, the pharmaceutical composition is for administration at a dose of 4 mg AXN-001 or a pharmaceutically active salt thereof by subcutaneous administration. Preferably, the pharmaceutical composition is for administration at a dose of 8 mg AXN-001 or a pharmaceutically active salt thereof by subcutaneous administration. Preferably, the pharmaceutical composition is for administration at a dose of 16 mg AXN-001 or a pharmaceutically active salt thereof by subcutaneous administration. Preferably, the pharmaceutical composition is for administration at a dose of 32 mg AXN-001 or a pharmaceutically active salt thereof by subcutaneous administration.

According to a further aspect of the invention, there is provided the use in the manufacture of a medicament for the treatment of acute migraine, of 1 mg to 18 mg of a compound of formula (I) or a pharmaceutically active salt thereof: 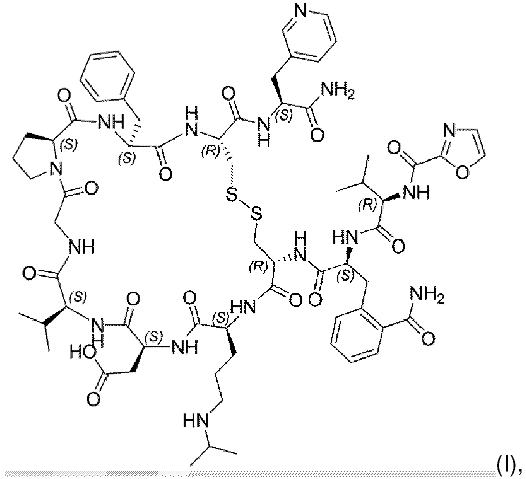 wherein the concentration of the compound of formula (I) or pharmaceutically active salt thereof is 2 mg/mL to 36 mg/mL.

According to a further aspect of the invention, there is provided a method of treatment of acute migraine comprising a step of administration to a patient in need thereof a pharmaceutical composition comprising 1 mg to 18 mg of a compound of formula (I) or a pharmaceutically active salt thereof: 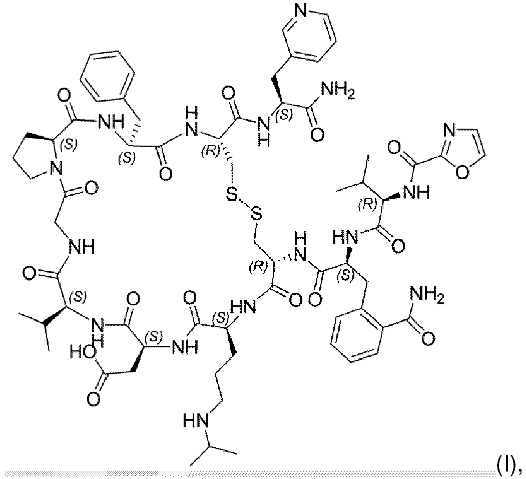 wherein the concentration of the compound of formula (I) or pharmaceutically active salt thereof is 2 mg/mL to 36 mg/mL.

According to a further aspect of the invention, there is provided a method of manufacturing a pharmaceutical composition comprising 1 mg to 18 mg of a compound of formula (I) or a pharmaceutically active salt thereof: 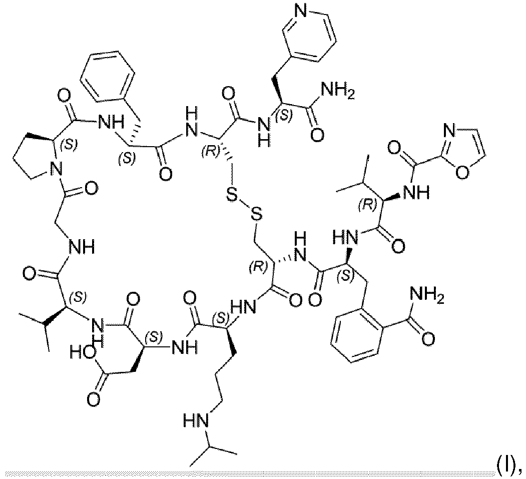 wherein the concentration of the compound of formula (I) or pharmaceutically active salt thereof is 2 mg/mL to 36 mg/mL.

Optionally, the method of manufacturing the pharmaceutical composition of 32 mg/mL ANX-001 and other concentrations comprises the steps of:
1) Preparing formulation buffer (10 mM Sodium Acetate, 45 mg/mL Mannitol, pH 5.0)
2) Formulating 32 mg/mL AXN-001 in formulation buffer
   a. adding approximately 12 mL formulation buffer
   b. adding 480 mg of AXN-001 peptide (content and purity adjusted) and mixing until peptide is dissolved
   c. adjusting pH of the formulation to pH 5.0 ±0.1 with hydrochloric acid or sodium hydroxide solution
   d. adding additional formulation buffer to the reaction mixture to final volume of 15mL and mixing.
3) Formulations of more dilute concentrations of AXN-001 may be prepared by appropriate dilution of the 32 mg/mL formulation in formulation buffer:
   a. 16 mg/mL AXN-001 may be prepared by adding 8 mL of the 32 mg/mL formulation from step 2 to 8 mL of formulation buffer.
   b. 8 mg/mL AXN-001 may be prepared by adding 4 mL of the 32 mg/mL formulation from step 2 to 12 mL of formulation buffer
   c. 4 mg/mL AXN-001 may be prepared by adding 2 mL of the 32 mg/mL formulation from step 2 to 14 mL of formulation buffer
   d. 2 mg/mL AXN-001 may be prepared by adding 1 mL of the 32 mg/mL formulation from step 2 to 15 mL of formulation buffer.
4) Filtering the formulation with a 0.2 µm polyether sulfone membrane filter into a sterile syringe
5) Storing at 2-8 °C.

Also provided is a method of treating a patient by administering a dose of AXN-001 or a pharmaceutically active salt thereof by subcutaneous administration. Preferably, a dose of 1 mg AXN-001 or a pharmaceutically active salt thereof is administered to the patient by subcutaneous administration. Preferably, a dose of 2 mg AXN-001 or a pharmaceutically active salt thereof is administered to the patient by subcutaneous administration. Preferably, a dose of 4 mg AXN-001 or a pharmaceutically active salt thereof is administered to the patient by subcutaneous administration. Preferably, a dose of 8 mg AXN-001 or a pharmaceutically active salt thereof is administered to the patient by subcutaneous administration. Preferably, a dose of 16 mg AXN-001 or a pharmaceutically active salt thereof is administered to the patient by subcutaneous administration.

Features described above in relation to any one aspect of the disclosure are equally applicable, mutatis mutandis, to every other aspect of the disclosure.

### Detailed Description

### Drug substance

AXN-001 is a 11-mer monocyclic, branched peptide, which may be isolated as a hydrochloride salt. The drug substance used in the development of the pharmaceutical composition and for the clinical studies was prepared by solid phase peptide synthesis. Screening and stability studies of the drug product confirm the compatibility of AXN-001 with the excipients used.

### Excipients

Mannitol is used as isotonicity agent. Sodium acetate trihydrate is used as buffering agent. Water for injection is used as solvent. Hydrochloric acid and sodium hydroxide may be used if required as pH adjusting agents.

### Method of Manufacturing

The method of manufacturing the pharmaceutical composition of 32 mg/mL ANX-001 and other concentrations comprises the steps of:
1) Preparing formulation buffer (10 mM Sodium Acetate, 45 mg/mL Mannitol, pH 5.0)
2) Formulating 32 mg/mL AXN-001 in formulation buffer
   a. adding approximately 12 mL formulation buffer
   b. adding 480 mg of AXN-001 peptide (content and purity adjusted) and mixing until peptide is dissolved
   c. adjusting pH of the formulation to pH 5.0 ±0.1 with hydrochloric acid or sodium hydroxide solution
   d. adding additional formulation buffer to the reaction mixture to final volume of 15mL and mixing.
3) Formulations of more dilute concentrations of AXN-001 may be prepared by appropriate dilution of the 32 mg/mL formulation in formulation buffer:
   a. 16 mg/mL AXN-001 may be prepared by adding 8 mL of the 32 mg/mL formulation from step 2 to 8 mL of formulation buffer.
   b. 8 mg/mL AXN-001 may be prepared by adding 4 mL of the 32 mg/mL formulation from step 2 to 12 mL of formulation buffer
   c. 4 mg/mL AXN-001 may be prepared by adding 2 mL of the 32 mg/mL formulation from step 2 to 14 mL of formulation buffer
   d. 2 mg/mL AXN-001 may be prepared by adding 1 mL of the 32 mg/mL formulation from step 2 to 15 mL of formulation buffer.
4) Filtering the formulation with a 0.2 µm polyether sulfone membrane filter into a sterile syringe
5) Storing at 2-8 °C.

### Development of pharmaceutical composition

Comparable peptides typically involve isotonic acetate buffer solutions at approximately pH 4.5. However, the Applicant surprisingly identified pharmaceutical compositions having higher pH values which in turn relate more closely to physiological pH levels. To achieve the higher pH values, an isotonic 10 mM phosphate buffer was used.

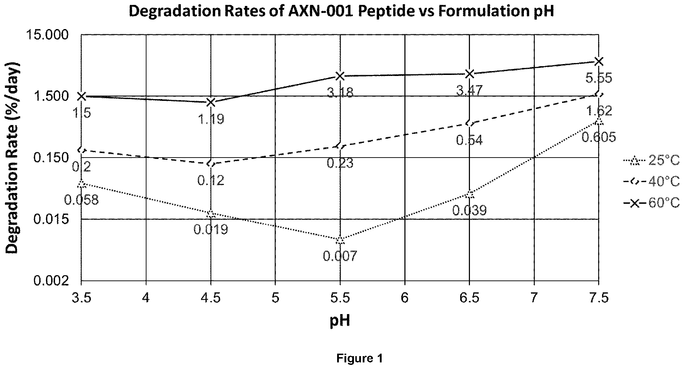

The degradation rates were then determined for 1 mg/mL solutions of AXN-001 at pH 3.5, 4.5, 5.5, 6.5, and 7.5, when kept at 2-8°C, 25°C, 40°C, and 60°C for 30 days. The degradation rates for various pH values and temperatures are shown in **Table 1** and **Figure 1** below.

**Table 1: Degradation rates**

| | Degradation Rates (%/day) | | | |
|---|---|---|---|---|
| pH | 2-8°C | 25°C | 40°C | 60°C |
| 3.5 | n.d. | 0.058 | 0.20 | 1.50 |
| 4.5 | n.d. | 0.019 | 0.12 | 1.19 |
| 5.5 | n.d. | 0.007 | 0.23 | 3.18 |
| 6.5 | n.d. | 0.039 | 0.54 | 3.47 |
| 7.5 | 0.02 | 0.605 | 1.62 | 5.55 |

| | | | | |
|---|---|---|---|---|
| Legend: n.d. = degradation too slow, not measureable | | | | |

Degradation rates at 2-8° C were not determined ("n.d.") because degradation could not be measured at thirty days (with the exception of pH 7.5).

The preliminary conclusions from the development of pharmaceutical composition were that:
- Pharmaceutical compositions at 40 and 60°C were the most stable at pH 4.5
- Pharmaceutical compositions at 25°C were the most stable at pH 5.5

A pH of 5 was selected as the target pH for the clinical trial formulation. The composition of the 32 mg/mL pharmaceutical composition is presented in **Table 2** below.

**Table 2: Composition of AXN-001 Clinical Formulation, 32 mg/mL**

| **Component** | **Amount per mL** |
|---|---|
| AXN-001 | 32.0 mg |
| Sodium acetate trihydrate | 1.36 mg |
| Mannitol | 45.0 mg |
| Hydrochloric acid | Adjust to pH 5.0 (if needed) |
| Sodium hydroxide | Adjust to pH 5.0 (if needed) |
| Sterile water for injection (Wifi) | q.s. to 1.0 mL |

For Phase I clinical studies, the 32 mg/mL pharmaceutical composition was dosed subcutaneously. The Phase I clinical trial was a randomised, double-blind, placebo-controlled, first-in-human study of the safety, tolerability and pharmacokinetics of AXN-001 in healthy volunteers under Protocol No. AXN-001-01 .

### Physiochemical and Biological Properties

The pH of the pharmaceutical composition was 5.0, which was ensured by measuring and adjusting pH as an in-process control. pH was also part of the release specification. Isotonicity of the drug product was achieved by addition of mannitol. The density was determined to be 1.015 g/cm³ (32 mg/mL).

### Clinical Trial

The pharmacokinetic objective of the clinical trial was to provide pharmacokinetic analysis of AXN-001 following single ascending subcutaneous doses of AXN-001. The study design included the following Cohorts:
- Cohort 1: Dose 1 mg (2 mg/mL formulation);
- Cohort 2: Dose 2 mg (4 mg/mL formulation);
- Cohort 3: Dose 4 mg (8 mg/mL formulation);
- Cohort 4: Dose 8 mg (16 mg/mL formulation);
- Cohort 5: Dose 16 mg (32 mg/mL formulation).

Participants were administered a single dose (0.5 mL) of AXN-001 or placebo via subcutaneous (SC) injection into the abdomen. Blood samples were collected at specified time points for pharmacokinetic analysis (5,10,15,20,30,45,60 minutes; 2,3,4,6,8,12,24 hours). Individual plasma concentrations of ANX-001 were determined and used for analyses.

Pharmacokinetic (PK) parameters of AXN-001 were determined using Phoenix WinNonlin version 8.3. All concentrations below the limit of quantification (BLQ) were set to zero for the purpose of the PK analysis. All AUC determinations were made using linear trapezoidal method. The following parameters were determined:
- Cₘₐₓ: Maximum observed concentration. as read directly from the observed data.
- Tₘₐₓ: Time to maximum concentration
- T_{10nM}: Time to 10 nM
- T_{30nM}: Time to 30 nM
- T_{100nM}: Time to 100 nM
- AUC₀₋ₜ: Area under the concentration-time curve from time zero to the time of the last measurable concentration, calculated using linear trapezoidal rule
- AUC_{0-inf}: Area under the concentration-time curve from time zero to infinity, calculated using linear trapezoidal rule
- %AUCₑₓₜᵣₐₚ: The portion of AUC determined by extrapolation
- t_{1/2}: Apparent terminal elimination half-life
- C_{L/F} or C_{L}: Total apparent body clearance
- V_{z/F} or V_{z}: Apparent volume of distribution
- λz: Terminal elimination rate constant

Table 3 summarizes the mean plasma concentrations of AXN-001 measured at various time points following a single subcutaneous dose.

**Table 3**

| | **Nominal time (h)** | | | | | | | | | | | | |
|---|---|---|---|---|---|---|---|---|---|---|---|---|---|
| | **0.08** | **0.17** | **0.25** | **0.33** | **0.50** | **0.75** | **1** | **2** | **3** | **4** | **6** | **8** | **12** |
| **Dose** | **Plasma Concentration AXN-001 (ng/mL)** | | | | | | | | | | | | |
| **1 mg** | 4.96 | 12.8 | 17.9 | 28.5 | 34.8 | 39.3 | 35.1 | 22.8 | 13.7 | 7.47 | 2.8 | 0 | 0 |
| **2 mg** | 11.3 | 21.6 | 31.5 | 40.4 | 55.7 | 62.6 | 60.2 | 38.1 | 21.3 | 11.4 | 4.79 | 0.713 | 0 |
| **4 mg** | 9.6 | 35.3 | 71.4 | 87.8 | 115 | 123 | 134 | 82.5 | 44.7 | 23.5 | 8.25 | 3.09 | |
| **8 mg** | 32.1 | 84 | 118 | 144 | 199 | 240 | 226 | 136 | 72 | 38.7 | 13.9 | 5.8 | 0.38 |
| **16 mg** | 27.4 | 83.4 | 139 | 183 | 294 | 355 | 380 | 277 | 170 | 96.8 | 44 | 23.7 | 9.2 |

Table 4 provides the summary mean plasma PK Parameters for Cohorts 1 to 5.

**Table 4**

| **Dose** | **Tmax (h)** | **Cmax (ng/mL)** | **AUC₀₋ₜ (h*ng/mL)** | **AUC_{0-inf} (h*ng/mL)** | **%AUCextrap (%)** | **CL/F (L/h)** | **Vz/F (L/h)** | **T_{1/2} (h)** | **λz (1/h)** |
|---|---|---|---|---|---|---|---|---|---|
| **1 mg** | 0.792 | 39.5 | 96.1 | 101 | 5.37 | 9.99 | 19.1 | 1.31 | 0.535 |
| **2 mg** | 0.792 | 65 | 159 | 167 | 4.98 | 12.6 | 24.6 | 1.35 | 0.528 |
| **4 mg** | 0.700 | 133 | 337 | 343 | 1.82 | 11.8 | 23.3 | 1.36 | 0.517 |
| **8 mg** | 0.750 | 244 | 585 | 594 | 1.58 | 13.5 | 28.7 | 1.47 | 0.481 |
| **16 mg** | 0.958 | 381 | 1230 | 1250 | 1.51 | 13.3 | 55.3 | 2.80 | 0.292 |

Table 5 shows the time required for blood levels to reach certain target concentrations of AXN-001 after subcutaneous administration.

**Table 5**

| **Dose (mg)** | **T10nM (min)** | **T30nM (min)** | **T100nM (min)** |
|---|---|---|---|
| 1 | 12 | 19* | |
| 2 | 9 | 21* | |
| 4 | 6 | 11 | 25* |
| 8 | 3 | 7 | 21 |
| 16 | 3 | 7 | 17 |

As shown in Table 5, all subjects at all doses were able to achieve plasma concentrations of 10nM AXN-001 in less than 15 minutes. All subjects in the 4, 8, and 16 mg cohort, 5 of 6 subjects in the 2 mg cohort, and 1 of 6 subjects in the 1 mg cohort achieved plasma concentrations of 30nM. All subjects in the 8 and 16 mg cohorts, and 2/5 of the 4 mg cohort, achieved 100nM plasma concentrations.

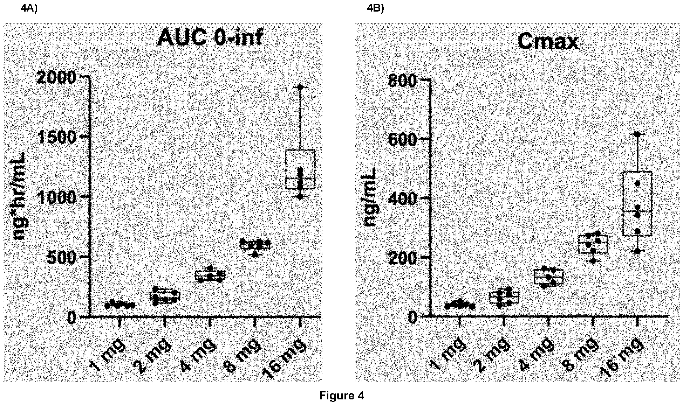

**Figure 4** relates to the summary of AXN-001 plasma concentrations. More specifically, **Figure 4A** relates to the summary plasma concentrations of AXN-001 following single ascending subcutaneous dose of AXN-001 for Cohort 1 to 5 on a linear scale and **Figure 4B** on a semi-log scale.

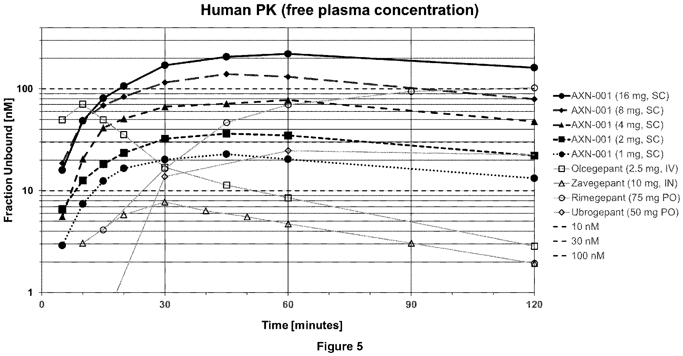

**Figure 5** shows the differentiated/superior PK profile of AXN-001. More specifically, the data shown in Figure 5 demonstrates that within 30 minutes, the AXN-001 much higher increase in free plasma concentration when compared with olcegepant, zavegepant, rimegepant and ubrogepant whilst maintaining a consistent free plasma concentration up to 120 minutes.

Summarising, the human PK analyses of subcutaneous injection of AXN-001 demonstrated:
- Faster, higher, and more consistent free plasma concentrations than any approved gepant
- Faster, higher, and more reliable efficacy than any approved gepant.

### List of Figures

Embodiments of the invention will now be described, by way of illustration only, with reference to the accompanying figures, in which:
Figure 1 is a plot relating to AXN-001 degradation rates

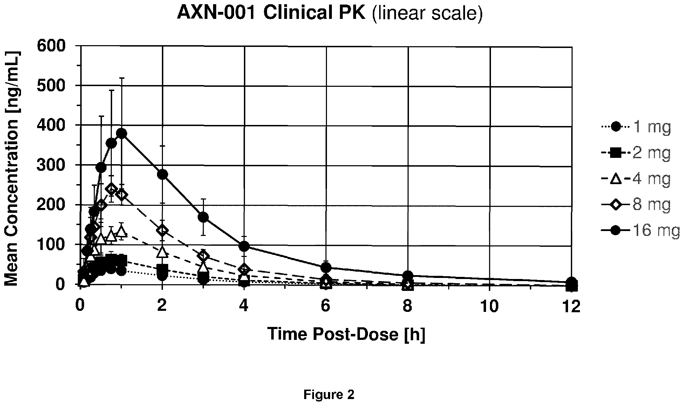

Figure 2 is plot relating to AXN-001 plasma concentrations

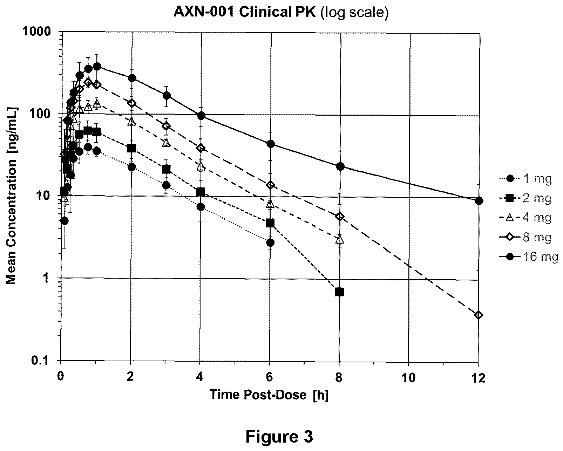

Figure 3 is plot relating to AXN-001 plasma concentrations
Figure 4a is plot relating to AXN-001 AUC₍₀₋ₜ₎
Figure 4b is plot relating to AXN-001 Cₘₐₓ
Figure 5 is a plot relating to PK profile of AXN-001

## Claims

1. A pharmaceutical composition comprising 1 mg to 18 mg of a compound of formula (I) or a pharmaceutically active salt thereof: 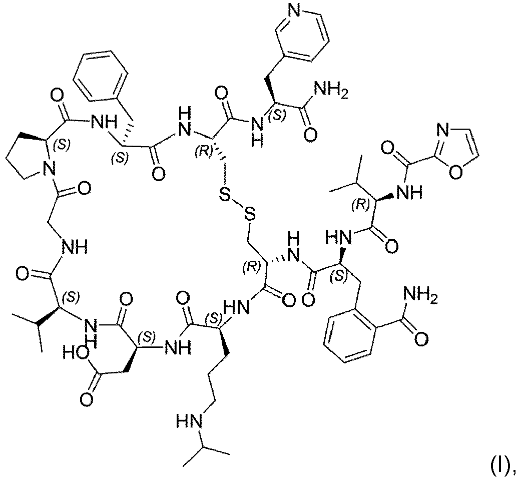 wherein the concentration of the compound of formula (I) or pharmaceutically active salt thereof is 2 mg/mL to 36 mg/mL.

2. The pharmaceutical composition according to claim 1 comprising 4 mg to 18 mg of a compound of formula (I) or a pharmaceutically active salt thereof, preferably 6 mg to 18 mg of a compound of formula (I) or pharmaceutically active salt thereof.

3. The pharmaceutical composition according to claim 1 comprising 1 mg to 6 mg of a compound of formula (I) or pharmaceutically active salt thereof, preferably 3 mg to 5 mg of a compound of formula (I) or pharmaceutically active salt thereof.

4. The pharmaceutical composition according to any preceding claim, wherein the pharmaceutical composition comprising the compound of formula (I) or an equivalent net peptide weight of a pharmaceutically active salt thereof:

5. The pharmaceutical composition according to claim 2, wherein the concentration of the compound of formula (I) or a pharmaceutically active salt thereof is 8 mg/mL to 36 mg/mL, preferably 12 mg/mL to 36 mg/mL.

6. The pharmaceutical composition according to claim 3, wherein the concentration of the compound of formula (I) or a pharmaceutically active salt thereof is 2 mg/mL to 12 mg/mL, preferably 6 mg/mL to 10 mg/mL.

7. The pharmaceutical composition according to any preceding claim, comprising:
(i) 1 mg of a compound of formula (I) or a pharmaceutically active salt thereof, wherein the concentration of the compound of formula (I) or a pharmaceutically active salt thereof is 2 mg/mL;
(ii) 2 mg of a compound of formula (I) or a pharmaceutically active salt thereof, wherein the concentration of the compound of formula (I) or a pharmaceutically active salt thereof is 4 mg/mL;
(iii) 4 mg of a compound of formula (I) or a pharmaceutically active salt thereof, wherein the concentration of the compound of formula (I) or a pharmaceutically active salt thereof is 8 mg/mL;
(iv) 8 mg of a compound of formula (I) or a pharmaceutically active salt thereof, wherein the concentration of the compound of formula (I) or a pharmaceutically active salt thereof is 16 mg/mL; or
(v) 16 mg of a compound of formula (I) or a pharmaceutically active salt thereof, wherein the concentration of the compound of formula (I) or a pharmaceutically active salt thereof is 32 mg/mL.

8. The pharmaceutical composition according to any preceding claim, wherein the composition is an injectable pharmaceutical composition.

9. The pharmaceutical composition according to any preceding claim, wherein the composition is aqueous.

10. The pharmaceutical composition according to any preceding claim, wherein the pH of the composition is 4.5 to 5.5.

11. The pharmaceutical composition according to any preceding claim, comprising a buffering agent, preferably wherein the buffering agent is sodium acetate trihydrate and/or comprising an isotonicity agent, preferably wherein the isotonicity agent is mannitol.

12. The pharmaceutical composition according to any preceding claim for use in the treatment of acute migraine.

13. The pharmaceutical composition according to any preceding claim, wherein the composition is for administration at a dose of 1 mg to 16 mg/subject.

14. The pharmaceutical composition according to any preceding claim, wherein the composition is administered subcutaneously.

15. A method of treatment of acute migraine comprising a step of administration to a patient in need thereof a composition according to any preceding claim.
